(19) Europäisches Patentamt
European Patent Office
Office européen des brevets

(11) Publication number: **0 041 827 B1**

(12) **EUROPEAN PATENT SPECIFICATION**

(45) Date of publication of patent specification: **21.03.84**

(51) Int. Cl.³: **C 07 C119/06**, A 61 K 31/195

(21) Application number: **81302467.6**

(22) Date of filing: **03.06.81**

(54) **A N-(benzylidene)aminomethyl-cyclohexane-carboxylic acid.**

(30) Priority: **04.06.80 JP 75055/80**
**24.02.81 JP 25990/81**

(43) Date of publication of application:
**16.12.81 Bulletin 81/50**

(45) Publication of the grant of the patent:
**21.03.84 Bulletin 84/12**

(84) Designated contracting states:
**BE CH DE FR GB IT LI NL SE**

(56) References cited:
**CHEMICAL ABSTRACTS, vol. 69, no. 19, 4th November 1968, page 7187, column 2, no. 76971n, Columbus, Ohio, USA**

(73) Proprietor: **KUREHA KAGAKU KOGYO KABUSHIKI KAISHA**
**9-11 Horidome-cho 1-chome Nihonbashi Chuo-ku Tokyo (JP)**

(72) Inventor: **Takita, Hitoshi**
**1-8 Toyotama-kita**
**Nerima-ku Tokyo (JP)**
Inventor: **Mukaida, Yutaka**
**No. 21 Daiichi-Kurehasoh 3-10-13**
**Nerima-ku Tokyo (JP)**
Inventor: **Noda, Sakuo**
**No. 103 Kureha Ohkubo-Shataku**
**3-26-1 Hyakunin-cho Shinjuku-ku Tykyo (JP)**
Inventor: **Kobayashi, Hidetoshi**
**2-21-2 Zenpukuji**
**Suginami-ku Tokyo (JP)**

(74) Representative: **Myerscough, Philip Boyd et al**
**J.A.Kemp & Co. 14, South Square Gray's Inn**
**London, WC1R 5EU (GB)**

**EP 0 041 827 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue St-Denis, 75001 Paris, France

A N-(benzylidene)aminomethyl-cyclohexane-carboxylic acid

This invention relates to a N-(benzylidene)aminomethyl-cyclohexane-carboxylic acid, to its production and to a pharmaceutical composition containing this N-(benzylidene)aminomethyl-cyclohexane-carboxylic acid.

The present invention provides 4-[N-(3',4'-dihydroxybenzylidene)aminomethyl] cyclohexane-1-carboxylic acid and salts and esters thereof. 4-[N-(3',4'-dihydroxybenzylidene)aminomethyl] cyclohexane-1-carboxylic acid (hereinafter referred to as the compound of the invention) is represented by the formula (I) :

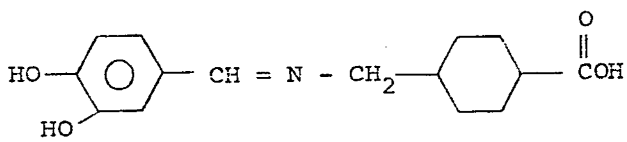

(I)

wherein the cyclohexane ring can be in the trans- or cis form. Salts of the compound of the invention include alkali and alkaline earth metal salts such as the sodium salt, potassium salt, calcium salt or magnesium salt, and substituted or unsubstituted ammonium salts. The ester is preferably a lower alkyl ester in which the alkyl group has 1 to 3 carbon atoms, such as methyl-, ethyl- or n- or isopropyl.

The compound of the invention or an ester or salt thereof is preferably prepared by reacting 3,4-dihydroxybenzaldehyde represented by the formula (II) :

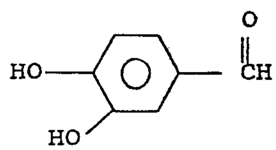

(II)

with 4-aminomethyl-cyclohexane-1-carboxylic acid represented by the formula (III) :

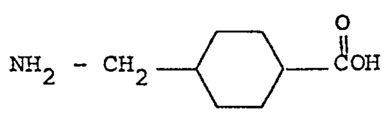

(III)

wherein the cyclohexane ring may be in the trans- or cis form, or an ester thereof, preferably a lower alkyl ester. A dehydration-condensation reaction occurs. The resulting compound of the invention can be converted into a salt or ester thereof or the resulting ester of the compound of the invention can be converted into the compound of the invention or a salt or another ester thereof.

The reaction of the compound of the formula (II) with the compound of the formula (III) or ester thereof is preferably carried out in an organic solvent at less than 150 °C, preferably at from 0 to 120 °C, under an atmosphere of an inert gas. At temperatures higher than 150 °C, the yield of the desired product is reduced because of various side reactions. Any organic solvent may be used for the reaction provided it does not participate in the reaction. A lower alcohol such as methanol or ethanol, benzene, toluene, dimethylformamide or acetonitrile is conventionally used for the solvent.

Since the reaction takes place with dehydration, the reaction is preferably carried out in the presence of a dehydrating agent or while removing water formed by the reaction by refluxing the solvent. An anhydrous form of a lower alcohol, such as anhydrous methanol or ethanol, can be used as the solvent and at the same time as the dehydrating agent. The compound of the invention can be isolated by treating the reaction mixture in a known manner after the reaction has finished.

A salt of the compound of the invention can be prepared by conventional methods involving neutralization of the compound of the invention by using a base such as a hydroxide, carbonate or bicarbonate of an alkali or alkaline earth metal, for example sodium, potassium, calcium or magnesium, or of ammonia or a primary-, secondary- or tertiary amine. For example, a sodium salt is obtained by neutralizing the compound of the invention with an alcoholic or aqueous solution of sodium hydroxide under an atmosphere of an inert gas at a temperature of less than 100 °C, usually from 0 to 50 °C.

The process mentioned above is only one way of preparing the compound of the invention. Other routes may be used.

The compound of the invention and salts and esters thereof show an inhibitory effect on platelet aggregation, polynuclear leukocyte migration, exudation of polynuclear leukocytes into an inflammatory site and proliferation of inflammatory granuloma, a prophylactic effect on adjuvant arthritis, an antineoplastic effect, a low acute toxicity, no lesions on the gastric mucosa and no mutagenicity, as will be shown in Examples. Accordingly, the compound of the invention is useful in therapy for treating, for example, inflammation, thrombosis, encephalorrhagia, hypertension, asthma or cancer, and especially

for treating chronic diseases such as rheumatism or systemic lupus erythematosus (SLE). The present invention therefore also provides a pharmaceutical composition comprising 4-[N-(3',4'-dihydroxybenzylidene)aminomethyl] cyclohexane-1-carboxylic acid or a pharmaceutically acceptable salt or ester thereof as active ingredient, together with a pharmaceutically acceptable carrier or adjuvant.

The composition of the invention can be administered perorally, rectally or by injection in various dosage forms. A mixture of two or more kinds of the compound of the invention and salts and esters thereof can be used. Other pharmaceutically active materials may be incorporated in the composition.

The dosage form of the composition may be as a tablet, sublingual tablet, powder, capsule, troch, aqueous or oily solution, suspension, emulsion, syrup, or aqueous or oily injection. Examples of the carrier incorporated in the composition include water, gelatin, lactose, starch, pectin, magnesium stearate, stearic acid, talc, vegetable oil, gum arabic, polyalkylene glycol, vaseline, sorbitan trioleate, polyoxyethylene-sorbitan monooleate, alkylphenol, aliphatic alcohol and polyvinylpyrrolidone. In the composition of the invention, if necessary, an edulcorant, flavor tinctorial agent, preservative, salt for osmoregulation or buffer, that is, a conventional pharmaceutical adjuvant, may be used.

The content of the compound of the invention or salt or ester thereof in a pharmaceutical composition may be suitably varied, for example, within a range of 0.01 %-100 % by weight of the composition, preferably 0.05 %-80 % by weight of the composition.

The pharmaceutical composition of the invention may be administered to a human or animal parenterally, for example, rectally, by injection (hypodermic, intramuscular or intravenous, or drip), preferably perorally (for example sublingually). A dose of the pharmaceutical composition of the invention can consist of $10^{-7}$ to $5 \times 10^{-4}$ kg (0.1 to 500 mg), preferably $5 \times 10^{-7}$ to $2 \times 10^{-4}$ kg (0.5 to 200 mg) per day per kilogram of body weight in the case of peroral administration to a human, and $10^{-8}$ to $2 \times 10^{-4}$ kg (0.01 to 200 mg), preferably $10^{-7}$ to $10^{-4}$ kg (0.1 to 100 mg) in the case of parenteral administration, administered one to four times a day. However the dose of the composition depends on, for example, age, the individual concerned and the condition of a disease of a human or animal. A dose outside the above-mentioned ranges may be used.

The following Examples illustrate the present invention.

## Example 1

Preparation of trans-4-[N-(3',4'-dihydroxybenzylidene)aminomethyl] cyclohexane-1-carboxylic acid :

$1.753 \times 10^{-3}$ Kg (1.753 g) (12.7 mmol) of 3,4-dihydroxybenzaldehyde was dissolved in $3 \times 10^{-5}$ $m^3$ (30 ml) of dehydrated and purified methanol. The resulting solution was added dropwise into $1.994 \times 10^{-3}$ kg (1.994 g) (12.7 mmol) of trans-4-aminomethyl-cyclohexane-1-carboxylic acid under an atmosphere of nitrogen. This mixed solution was refluxed for 3 hours while stirring. After cooling the reaction mixture to room temperature, the orange-yellow crystals that deposited were filtered off, washed with methanol and vacuum-dried. $2.9 \times 10^{-3}$ kg (2.9 g) of trans-4-[N-(3',4'-dihydroxybenzylidene)aminomethyl] cyclohexane-1-carboxylic acid was obtained in a yield of 82.7 %.

The characteristics of the compound obtained were as follows :

(1) melting point ; 222 °C with decomposition,

(2) elementary analysis :

| | C(%) | H(%) | N(%) |
|---|---|---|---|
| theoretical : | 64.97 | 6.91 | 5.05 |
| experimental : | 64.6 | 7.1 | 5.0 |

(3) nuclear magnetic resonance (NMR) spectrum in dimethyl sulfoxide ;

$\delta$ = 0.9-2.0 (9H, m), 2.1-2.2 (1H), 3.33 (2H, d), 6.73 (1H, d), 6.97 (1H, d), 7.19 (1H, s), 8.03 (1H, s),

(4) infrared absorption (IR) spectrum by KBr tablet method, as shown in Fig. 1 of the accompanying drawings.

## Example 2

Preparation of sodium trans-4-[N-(3',4'-dihydroxybenzylidene)aminomethyl] cyclohexane-1-carboxylate :

$10^{-3}$ Kg (1.00 g) (3.61 mmol) of trans-4-[N-(3',4'-dihydroxybenzylidene)aminomethyl] cyclohexane-1-carboxylic acid obtained in Example 1 was added to $3.6 \times 10^{-6}$ $m^3$ (3.6 ml) of 1N methanolic solution of sodium hydroxide, and dissolved while stirring for 20 to 30 min at room temperature under an atmosphere of nitrogen. After filtration, the methanol was evaporated off. $1.121 \times 10^{-3}$ Kg (1.121 g) of sodium trans-4-[N-(3',4'-dihydroxybenzylidene)aminomethyl] cyclohexane-1-carboxylate was obtained.

The IR spectrum (KBr tablet method) of the compound obtained is shown in Fig. 2 of the accompanying drawings. In the ultraviolet (UV) absorption spectrum in methanol the following two maxima were observed :

$\lambda$max : 271.5 m$\mu$ and 309 m$\mu$.

Example 3

Preparation of ethyl trans-4-[N-(3′,4′-dihydroxybenzylidene)aminomethyl] cyclohexane-1-carboxylate :

$8.35 \times 10^{-4}$ Kg (0.835 g) of 3,4-dihydroxybenzaldehyde and $1.2 \times 10^{-3}$ kg (1.2 g) of ethyl trans-4-aminomethyl-cyclohexane-1-carboxylate were dissolved in $2 \times 10^{-5}$ $m^3$ (20 ml) of methanol. The resulting solution was refluxed and reacted for one hour under an atmosphere of nitrogen. After leaving the reaction mixture at 0 °C overnight, the purple plate-crystals that deposited were filtered off and dried to obtain $1.525 \times 10^{-3}$ kg (1.525 g) of the ethyl ester of the compound of the invention.

The characteristics of the ethyl ester thus obtained were as follows :

(1) melting point ; 50-62 °C,
(2) elementary analysis ;

| | C(%) | H(%) | N(%) |
|---|---|---|---|
| theoretical : | 66.86 | 7.59 | 4.59 |
| experimental : | 66.2 | 7.7 | 4.3 |

(3) IR spectrum ; as shown in Fig. 3 of the accompanying drawings.

Example 4

Preparation of cis-4-[N-(3′,4′-dihydroxybenzylidene)aminomethyl] cyclohexane-1-carboxylic acid :

$3.95 \times 10^{-4}$ Kg (0.395 g) (2.86 mmol) of 3,4-dihydroxybenzaldehyde was refluxed in dehydrated and purified methanol to react it with $4.5 \times 10^{-4}$ kg (0.450 g) (2.86 mmol) of cis-4-aminomethyl-cyclohexane-1-carboxylic acid for one hour. After evaporating off the methanol under a reduced pressure from the uniform red-orange reaction mixture, $7.91 \times 10^{-4}$ kg (0.7910 g) of a yellow powder of cis-4-[N-(3′,4′-dihydroxybenzylidene)aminomethyl] cyclohexane-1-carboxylic acid was obtained.

The characteristics of the compound of the invention (cis form) thus obtained were as follows :

(1) melting point ; 121-129 °C,
(2) NMR in dimethylsulfoxide ;
$\delta$ = 1.02-1.84 (9H, m), 2.0-2.1 (1H), 3.35 (2H, d), 6.82 (1H, d), 6.97 (1H, d), 7.20 (1H, s), 8.04 (1H, s).

Example 5

Inhibitory effect on platelet aggregation and polynuclear leukocyte migration, and acute toxicity :

The pharmacological activity and acute toxicity of sodium salts of the compound of the invention were examined. The specimens examined are as follows :

Specimen I ; Sodium trans-4-[N-(3′,4′-dihydroxybenzylidene)aminomethyl] cyclohexane-1-carboxylate obtained in Example 2, and

Specimen II ; Sodium salt of cis-4-[N-(3′,4′-dihydroxybenzylidene)aminomethyl] cyclohexane-1-carboxylic acid obtained in Example 4.

Comparative Specimen III ; indomethacin, and IV ; homochlorcyclizine.

Comparative Specimens III and IV, having similar pharmacological activity to the compound of the invention, are commercially available in Japan.

The examination was carried out according to the methods described below :

(1) Examination method for inhibitory effect on platelet aggregation :

Rabbit platelet-rich plasma (PRP) was used for Aggregation Tests. PRP was prepared by centrifugation of blood collected from the ear vein of Rabbit and diluted into number-concentration 300,000/$10^{-9}$ $m^3$ (μl) with platelet-poor plasma. Platelet aggregation was induced by Sodium Arachidonate and monitored (measured) with a four channel platelet aggregation Tracer (PAT-4A (Niko Bioscience Co., Japan). An Aggregation Tracer tube containing $2.3 \times 10^{-7}$ $m^3$ (230 μl) PRP was preincubated at 37 °C for 5 min with each Specimen.

(2) Examination method for inhibitory effect on polynuclear leukocyte migration :

Rat's polynuclear leukocyte migration was examined by the Boyden method (refer to Japanese J. Clin. Med., *27* (9), 172 (1969)) as follows. A mixture of 10 parts by volume of culture filtrate of *E. coli* and one part by volume of serum was incubated at 37 °C for one hour and diluted to 5-times its original volume with a physiological salt solution. The resulting mixture was used as an attractant. After

preincubating the polynuclear leukocyte suspension with each specimen for 10 min, migration of polynuclear leukocytes was examined.

(3) Examination method for acute toxicity :

Acute toxicity was examined by perorally administering an aqueous solution of each Specimen to female JCL-ICR mice 5 to 6 weeks after birth.

The results are shown in Table 1.

| Specimen | Platelet aggregation $ID_{100}$ (μM)[1] | Inhibition rate of polynuclear leukocyte migration (%)[2] | | | |
|---|---|---|---|---|---|
| | | 1μM | 10μM | 100μM | 1000μM |
| I | 13-20 | 15 | 44 | 81 | 100 |
| II | 120 | | | 45.9 | |
| III | 0.5-1 | 0 | 0 | 25 | 79 |
| IV | ≥1000 | 15 | 48 | 80 | 100 |

Table 1 continued

| Specimen | Acute toxicity $LD_{50}$ ($10^{-6}$ kg/kg), (mg/kg)[3] |
|---|---|
| I | > 3000 [4] |
| II | > 3000 |
| III | 30 |
| IV | 350 |

Notes :

(1) The minimum concentration (μM) of each Specimen for completely inhibiting (100 %) the platelet aggregating effect of sodium arachidonate (tested at 400 μM).

(2) The average value of the inhibition rate (%) of polynuclear leukocyte migration at each concentration (μM) of each Specimen.

(3) Median lethal dose ($LD_{50}$), that is, the dose causing the death of 50 % of the mice, in units of $10^{-6}$ kg (mg) per kilogram of body weight of a mouse.

As shown in Table 1, the sodium salts of the compound of the invention has an inhibitory effect on platelet aggregation and polynuclear leukocyte migration, and a low acute toxicity. Accordingly, the salts can be used as an anti-inflammatory agent and in treating various other diseases.

## Example 6

Inhibitory effect on polynuclear leukocyte exudation :

The inhibitory effect of sodium trans-4-[N-(3′,4′-dihydroxybenzylidene)aminomethyl] cyclohexane-1-carboxylate on the exudation of polynuclear leukocytes into a site of inflammation was examined by using male rats (Donryu) with a body weight of about $1.5 \times 10^{-1}$ kg (150 g) and the Granuloma CMC pouch method (refer to Yakugaku Zasshi, *88,* 1472 (1968)).

After shearing the rat on the dorsum about $5 \times 10^{-2}$ m (5 cm) in diameter, $5 \times 10^{-6}$ $m^3$ (5 ml) of air was hypodermically injected into the site to form a pouch. After 24 hours, $5 \times 10^{-6}$ $m^3$ (5 ml) of an aqueous 2 % (W/V) solution of CMC sodium salt at 37 °C was injected into the pouch. At the same time an aqueous solution of the compound of the invention (sodium salt) was administered perorally to the rat at a dose of $5 \times 10^{-5}$, $10^{-4}$ and $2 \times 10^{-4}$ kg/kg (50, 100 and 200 mg/kg). For comparison, indomethacin was administered orally to the rat at a dose of $10^{-5}$ kg/kg (10 mg/kg) in the form of an aqueous suspension.

After the injection of CMC, $5 \times 10^{-7}$ $m^3$ (0.5 ml) samples of the liquid in the pouch were collected as time passed by and stained. Then the number of polynuclear leukocytes which had exuded into the pouch

was counted. The result is shown in Fig. 4 of the accompanying drawings. As shown in Fig. 4, the compound of the invention (sodium salt) can cause variation in the exudation of polynuclear leukocytes depending on its dose.

In addition, ethyl trans-4-[N-3',4'-dihydroxybenzylidene)aminomethyl] cyclohexane-1-carboxylate was examined in the same manner. The ester also inhibited the exudation of polynuclear leukocytes.

## Example 7

Inhibitory effect on the proliferation of inflammatory granuloma :

The inhibitory effect of sodium trans-4-[N-(3',4'-dihydroxybenzylidene)aminomethyl] cyclohexane-1-carboxylate on the proliferation of inflammatory granuloma was examined by using male rats (Donryu) 5 weeks after birth and the method of Fujimura *et al.* (refer to Pharmacometrics, *19* (3), 329 (1980)).

A sheet of filter paper of $1.3 \times 10^{-2}$ m (13 mm) in diameter and $2.6 \times 10^{-4}$ (0.26 mm) in thickness was immersed in an aqueous 2 % solution of CMC containing $10^{-1}$ kg/m$^3$ (0.1 mg/ml) of dihydroxystreptomycin and penicillin (million units). Thus treated sheet was inserted hypodermically on the dorsum of an etherized rat. After palinesthesia, the compound of the invention (sodium salt) was administered perorally once a day for 8 days at a dose of $2.5 \times 10^{-5}$, $5.0 \times 10^{-5}$ and $10^{-4}$ kg/kg/day (25, 50 and 100 mg/kg/day). For comparison, indomethacin was administered in the same manner at a dose of $3 \times 10^{-6}$ lg/kg/day (3 mg/kg/day). The body weight gain, the weight of granuloma and the lesions on the gastric mucosa were examined on the nineth day.

The results are shown in Table 2.

Table 2

| Pharmaceutical | Dose $10^{-6}$kg/kg/day (mg/kg/day) | Number of rats used | Granuloma | | Body Weight gain (%) | Lesions on stomachal mucous membrane |
|---|---|---|---|---|---|---|
| | | | Dry weight: $10^{-6}$ kg (mg) | Inhibitory rate (%) | | |
| Control[1)] | 0 | 4 | 330.2±73.9 | - | 29 | none |
| Compound of the invention (sodium salt) | 25 | 4 | 155.6±13.8 | 52.9[2)] | 28 | none |
| | 50 | 4 | 118.8±46.0 | 64.0[2)] | 32 | none |
| | 100 | 5 | 73.6±31.3 | 77.7[2)] | 37 | none |
| Indomethacin | 3 | 5 | 160.5±73.1 | 51.4[3)] | 23 | petechiae[4)] |

Notes :
1) The compound of the invention (sodium salt) is not administered,
2) $P < 0.01$, P denotes the limit of statistical confidence,
3) $P < 0.05$,
4) Petechiae were observed in two rats.

As shown in Table 2, the compound of the invention (sodium salt) effectively inhibits the proliferation of inflammatory granuloma while depending on the dose administered, and its administration does not inhibit the weight gain. Furthemore, no side effects, such as lesions on the mucous membrane of the stomach which can be seen when using conventional non-steroidal anti-inflammatory agents, were observed.

## Example 8

Lesions on the gastric mucosa :

Conventional anti-inflammatory agents may often cause lesions on the gastric mucosa as a side-effect. The possibility of the compound of the invention causing such lesions was examined by using the method of Fujimura *et al.* as in Example 7.

After a female JCL-SD rat had fasted for 24 hours, sodium trans-4-[N-(3',4'-dihydroxybenzylidene)-aminomethyl] cyclohexane-1-carboxylate was administered perorally to the rat. After 3 hours, the rat was slaughtered. Then the stomach was enucleated, and the length ($10^{-3}$ m) (mm) of the lesions on the gastric

mucosa was measured by the method of Okabe *et al.* (refer to Pharmacometrics, *18* (1), 1 (1979)). The total sum of the lengths was used as the Ulcer Index. For comparison, indomethacin was administered perorally at a dose of $3 \times 10^{-5}$ kg/kg (30 mg/kg). The results are shown in Table 3.

Table 3

| Pharmaceutics | Dose: $10^{-6}$kg/kg (mg/kg) | Number of rats | Ulcer Index |
|---|---|---|---|
| Control 1) | 0 | 2 | 0 |
| Compound of the invention (sodium salt) 2) | 200 | 2 | 0 |
| | 500 | 3 | 0 |
| Indomethacin 3) | 30 | 3 | 30.5 |

Notes :
1) The compound of the invention (sodium salt) is not administered.
2) Administered perorally in the form of an aqueous solution.
3) Administered perorally in the form of an aqueous suspension.

As shown in Table 3, the compound of the invention (sodium salt) even at as high a dose as $5 \times 10^{-4}$ kg/kg (500 mg/kg), did not cause lesions on the mucous membrane of the stomach.

Example 9

Prophylactic effect on adjuvant arthritis :

The prophylactic effect of the compound of the invention on adjuvant arthritis was examined by using six female JCL-SD rats 7 weeks after birth as one group and the conventional method (refer to Fujihira *et al.*, Pharmacometrics, 5 (2), 169-183, (1971)).

Freund's complete adjuvant (6 kg/m$^3$) (0.6 mg/0.1 ml) was inoculated into the right hind paw of an etherized rat. After palinesthesia, sodium trans-4-[N-(3',4'-dihydroxybenzylidene)aminomethyl] cyclohexane-1-carboxylate was administered perorally once a day for 24 continuous days. The change in the volume of the hind paw and in the body weight due to adjuvant arthritis was examined as time passed by. For comparison, a steroid (prednisolone) was administered perorally in the same manner.

The results are shown in Figs 5 and 6 of the accompanying drawings. In Fig. 5, « blank » denotes no inoculation of the adjuvant. As shown in Figs 5 and 6, the compound of the invention (sodium salt) effectively prevented the adjuvant arthritis without any reduction in the body weight of the rat which is often seen when a steroid is administered.

In addition, the rat was slaughtered in the 25th day from the inoculation of the adjuvant and the wieght of each organ of the rat was measured. No involution of the thymus was observed in the rats to which the compound of the invention (sodium salt) had been administered. There was a significant involution of the thymus in the rats to which the steroid (prednisolone) has been administered. Accordingly, the compound of the invention (sodium salt) is usable for treating chronic diseases such as rheumatism.

Example 10

Antineoplastic effect :

The antineoplastic effect of the compound of the invention was examined as follows :

$1 \times 10^6$ Cells of Sarcoma-180 were implanted into the infra-axillary site of ICR-JCL mice. 24 Hours after implantation, a solution of sodium trans-4-[N-(3',4'-dihydroxybenzylidene)aminomethyl] cyclohexane-1-carboxylate dissolved into sterilized and purified water for injection was administered perorally once a day for continuous 21 days at a dose of $10^{-4}$ and $5 \times 10^{-4}$ kg/kg (100 and 500 mg/kg). On the 22nd day after implantation, the neoplastic tuberculum was enucleated. The inhibition rate (I. R. in %) was calculated according to the following formula :

$$(1 - T/e) \times 100 = \text{I. R.} \quad (\%)$$

wherein T and e denotes the mean weights of neoplasm of the groups to which the compound of the

invention (sodium salt) had been administered and the control groups, respectively. The I. R. values were 55.8 and 45.3 % at a dose of $10^{-4}$ kg (100 mg) and 5 × $100^{-4}$ kg (500 mg) of the compound of the invention (sodium salt), respectively.

Example 11

Mutagenicity :

The mutagenicity of the compound of the invention was examined by Rec-assay.

*Bacillus subtilis* M 45 (a strain deficient in recombinational repair) and *B. subtilis* H 17 (a strain retaining recombinational repair) were streaked onto B-II agar culture so that the start points of the two streaks did not overlap. The B-II agar culture was prepared by dissolving $10^{-2}$ kg (10 g) of meat extract, $10^{-2}$ kg (10 g) of polypeptone, 5 × $10^{-3}$ kg (5 g) of sodium chloride and 1.5 × $10^{-2}$ kg (15 g) of agar in $10^{-3}$ $m^3$ (1 000 ml) of distilled water and adjusting the pH to 7.0.

As soon as a solution of sodium trans-4-[N-(3',4'-dihydroxybenzylidene)aminomethyl] cyclohexane-1-carboxylate was absorbed into a circular filter paper (disc) of 8 × $10^{-3}$ m (8 mm) in diameter, the start points of the two streaks were covered with the filter paper. After incubating the strains at 37 °C for a night, the length of the growth inhibition zone was measured. Kanamycin was used as a negative control and mitomycin was used as a positive control. The results are shown in Table 4.

Table 4

| Pharmaceutics | Concentration ($10^{-9}$ kg/disc) (μg/disc) | Growth inhibition Zone: $10^{-3}$m (mm) | | |
|---|---|---|---|---|
| | | M45 | H17 | Difference |
| Compound of the invention (sodium salt) | 100 | 0 | 0 | 0 |
| | 500 | 0 | 0 | 0 |
| | 1,000 | 0 | 0 | 0 |
| | 5,000 | 3 | 3 | 0 |
| Kanamycin | 10 | 6 | 5 | 1 |
| | 100 | 10 | 10 | 0 |
| Mitomycin | 0.05 | 4 | 0 | 4 |
| | 0.50 | 15 | 9 | 6 |

As shown in Table 4, the compound of the invention (sodium salt) showed no mutagenicity, and has been found to have a high safety.

From the results of Examples 5 to 11, the compound of the invention has been found to show very specific pharmacological activities, and exhibit no toxicity and no side-effects. Accordingly the compound of the invention can be used in a pharmaceutical composition which is safe and can be employed in combating or preventing various diseases.

**Claims**

1. 4-[N-(3',4'-dihydroxybenzylidene)aminomethyl] cyclohexane-1-carboxylic acid or a salt or an ester thereof.

2. A compound according to claim 1, which is trans-4-[N-(3',4'-dihydroxybenzylidene)aminomethyl] cyclohexane-1-carboxylic acid.

3. A compound according to claim 1, which is sodium trans-4-[N-(3',4'-dihydroxybenzylidene)aminomethyl] cyclohexane-1-carboxylate.

4. A compound according to claim 1, which is ethyl trans-4-[N-(3',4'-dihydroxybenzylidene)aminomethyl] cyclohexane-1-carboxylate.

5. A compound according to claim 1, which is cis-4-[N-(3',4'-dihydroxybenzylidene)aminomethyl] cyclohexane-1-carboxylic acid.

6. A process for preparing 4-[N-(3',4'-dihydroxybenzylidene)aminomethyl] cyclohexane-1-carboxylic acid or a salt or an ester thereof, which process comprises reacting 3,4-dihydroxybenzaldehyde with

4-aminomethyl-cyclohexane-1-carboxylic acid or an ester thereof, and optionally either converting the resulting 4-[N-(3',4'-dihydroxybenzylidene)aminomethyl] cyclohexane-1-carboxylic acid into a salt or ester thereof or converting the resulting ester of 4-[N-(3',4'-dihydroxybenzylidene)aminomethyl] cyclohexane-1-carboxylic acid into 4-[N-3',4'-dihydroxybenzylidene)aminomethyl] cyclohexane-1-carboxylic acid or a salt or another ester thereof.

7. A pharmaceutical composition comprising 4-[N-(3',4'-dihydroxybenzylidene)aminomethyl] cyclohexane-1-carboxylic acid or a pharmaceutically acceptable salt or ester thereof as active ingredient, together with a pharmaceutically acceptable carrier or adjuvant.

8. A composition according to claim 7 which contains a compound claimed in any one of claims 2 to 5.

9. A composition according to claim 7 or 8 which contains 4-[N-(3',4'-dihydroxybenzylidene)aminomethyl] cyclohexane-1-carboxylic acid or a pharmaceutically acceptable salt or ester thereof which has been prepared by a process as claimed in claim 6.

**Ansprüche**

1. 4-[N-(3',4'-Dihydroxybenzyliden)aminomethyl]-cyclohexane-1-carboxylsäure oder ein Salz oder ein Ester hiervon.

2. Verbindung nach Anspruch 1, dadurch gekennzeichnet, daß sie eine trans-4-[N-(3',4'-Dihydroxybenzyliden)aminomethyl]-cyclohexan-1-carboxylsäure ist.

3. Verbindung nach Anspruch 1, dadurch gekennzeichnet, daß sie ein Natriumsalz der trans-4-[N-(3', 4'-Dihydroxybenzyliden)aminomethyl]-cyclohexan-1-carboxylsäure ist.

4. Verbindung nach Anspruch 1, dadurch gekennzeichnet, daß sie ein Äthylester der trans-4-[N-(3', 4'-Dihydroxybenzyliden)aminomethyl]-cyclohexan-1-carboxylsäure ist.

5. Verbindung nach Anspruch 1, dadurch gekennzeichnet, daß sie eine vis-4-[N-(3',4'-Dihydroxybenzyliden)aminomethyl]-cyclohexan-1-carboxylsäure ist.

6. Verfahren zur Herstellung der 4-[N-(3',4'-Dihydroxybenzyliden)aminomethyl]-cyclohexan-1-carboxylsäure oder eines Salzes oder eines Esters hiervon, dadurch gekennzeichnet, daß 3,4-Dihydroxybenzaldehyd mit 4-Aminomethyl-cyclohexan-1-carboxylsäure oder einem Ester hiervon umgesetzt wird und gegebenenfalls entweder die erhaltene 4-[N-(3',4'-Dihydroxybenzyliden)aminomethyl]-cyclohexan-1-carboxylsäure in eines ihrer Salze oder Ester oder der erhaltene Ester der 4-[N-(3',4'-Dihydroxybenzyliden)aminomethyl]-cyclohexan-1-carboxylsäure in die 4-K-(3',4'-Dihydrobenzyliden)aminomethyl]-cyclohexan-1-carboxylsäure oder ein Salz oder einen anderen ihrer Ester umgewandelt wird.

7. Pharmazeutische Zusammensetzung, dadurch gekennzeichnet, daß sie als Wirkstoff 4-[N-(3',4'-Dihydroxybenzyliden)aminomethyl]-cyclohexan-1-carboxylsäure oder ein pharmazeutisch verträgliches Salz oder einen Ester hiervon zusammen mit einem pharmazeutisch verträglichen Träger- oder Hilfsstoff enthält.

8. Zusammensetzung nach Anspruch 7, dadurch gekennzeichnet, daß sie eine Verbindung nach einem der vorhergehenden Ansprüche 2 bis 5 enthält.

9. Zusammensetzung nach Anspruch 7 oder 8, dadurch gekennzeichnet, daß sie eine 4-[N-(3',4'-Dihydroxybenzyliden)aminomethyl]-cyclohexan-1-carboxylsäure oder ein pharmazeutisch verträgliches Salz oder Ester hiervon enthält, die durch ein Verfahren nach Anspruch 6 erhalten wurden.

**Revendications**

1. Acide 4-[N-(3',4'-dihydroxybenzylidène)aminométhyl] cyclohexane-1-carboxylique ou un sel ou un ester de celui-ci.

2. Composé suivant la revendication 1, caractérisé en ce qu'il est l'acide trans-4-[N-(3',4'-dihydroxybenzylidène)aminométhyl] cyclohexane-1-carboxylique.

3. Composé suivant la revendication 1, caractérisé en ce qu'il est le trans-4-[N-(3',4'-dihydroxybenzylidène)aminométhyl] cyclohexane-1-carboxylate de sodium.

4. Composé suivant la revendication 1, caractérisé en ce qu'il est le trans-4-[N-(3',4'-dihydroxybenzylidène)aminométhyl] cyclohexane-1-carboxylate d'éthyle.

5. Composé suivant la revendication 1, caractérisé en ce qu'il est l'acide cis-4-[N-(3',4'-dihydroxybenzylidène)aminométhyl] cyclohexane-1-carboxylique.

6. Procédé de préparation de l'acide 4-[N-(3',4'-dihydroxybenzylidène)aminométhyl] cyclohexane-1-carboxylique ou d'un sel ou d'un ester de celui-ci, caractérisé en ce qu'il consiste à faire réagir le 3,4-dihydrobenzaldéhyde avec l'acide 4-aminométhyl-cyclohexane-1-carboxylique ou un ester de celui-ci et, éventuellement, soit à transformer l'acide 4-[N-(3',4'-dihydrobenzylidène)aminométhyl] cyclohexène-1-carboxylique en un sel ou un ester de celui-ci ou à transformer l'ester qui en résulte de l'acide 4-[N-(3',4'-dihydroxybenzylidène)aminométhyl] cyclohexane-1-carboxylique en acide 4-[N-(3',4'-dihydrobenzylidène)aminométhyl] cyclohexane-1-carboxylique ou un sel ou un autre ester de celui-ci.

7. Composition pharmaceutique comprenant l'acide 4-[N-(3',4'-dihydroxybenzylidène)aminomé-

thyl] cyclohexane-1-carboxylique ou un sel pharmaceutiquement acceptable ou un ester de celui-ci en tant qu'ingrédient actif en même temps qu'un véhicule ou un adjuvant pharmaceutiquement acceptable.

8. Composition selon la revendication 7, caractérisée en ce qu'elle comprend un composé revendiqué dans l'une quelconque des revendications 2 à 5.

9. Composition suivant la revendication 7 ou 8, caractérisée en ce qu'elle contient de l'acide 4-[N-(3',4'-dihydroxybenzylidène)aminométhyl] cyclohexane-1-carboxylique ou un sel pharmaceutiquement acceptable ou un ester de celui-ci qui a été préparé par un procédé tel que revendiqué dans la revendication 6.

FIG.1

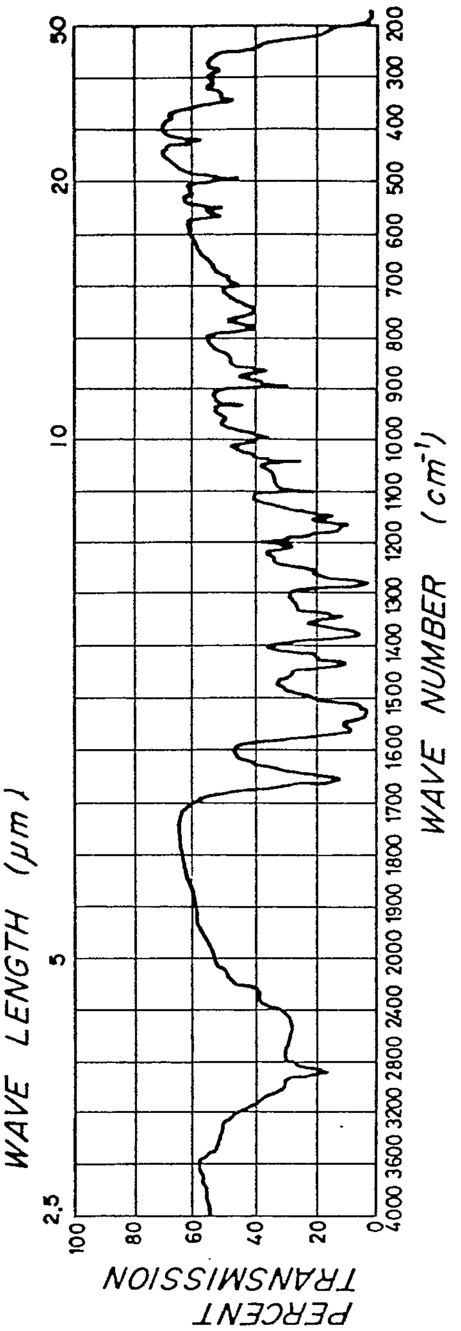
WAVE LENGTH (μm)
WAVE NUMBER $(cm^{-1})$
PERCENT TRANSMISSION

FIG.2

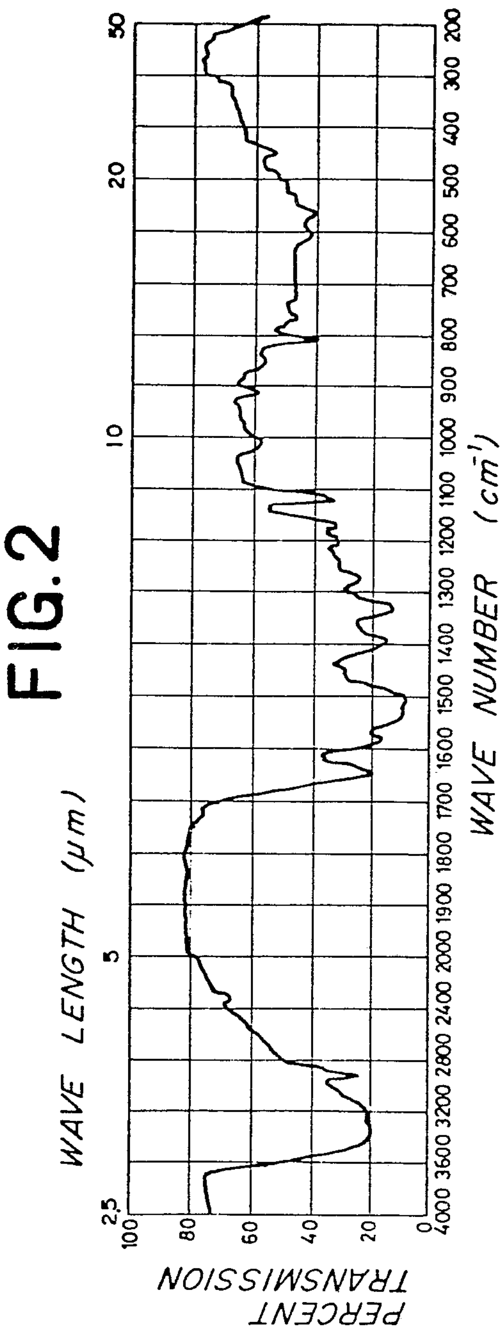
WAVE LENGTH (μm)
WAVE NUMBER $(cm^{-1})$
PERCENT TRANSMISSION

FIG.3

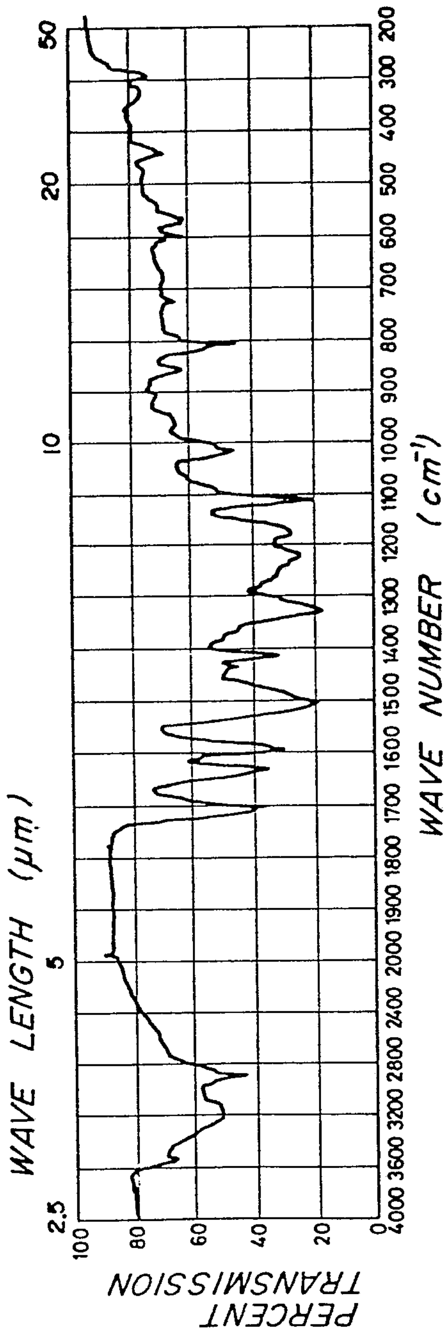

WAVE LENGTH (μm)
2.5
5
10
20
50
PERCENT TRANSMISSION
100
80
60
40
20
0
4000 3600 3200 2800 2400 2000 1900 1800 1700 1600 1500 1400 1300 1200 1100 1000 900 800 700 600 500 400 300 200
WAVE NUMBER (cm-1)

FIG. 4

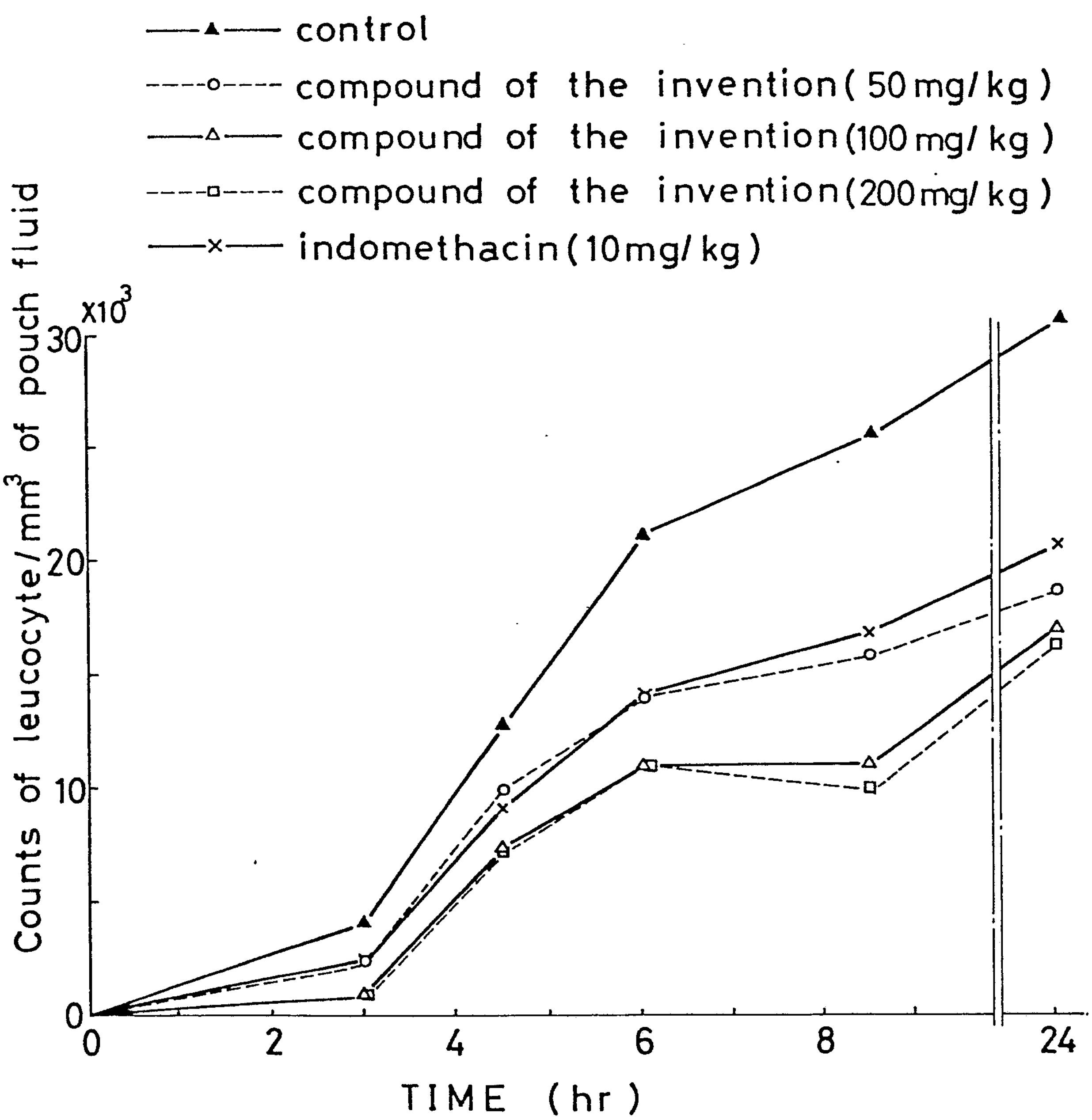
—▲— control
----○---- compound of the invention (50 mg/kg)
—△— compound of the invention (100 mg/kg)
----□---- compound of the invention (200 mg/kg)
—×— indomethacin (10 mg/kg)
Counts of leucocyte/mm3 of pouch fluid
X10³
30
20
10
0
0
2
4
6
8
24
TIME (hr)

FIG.5

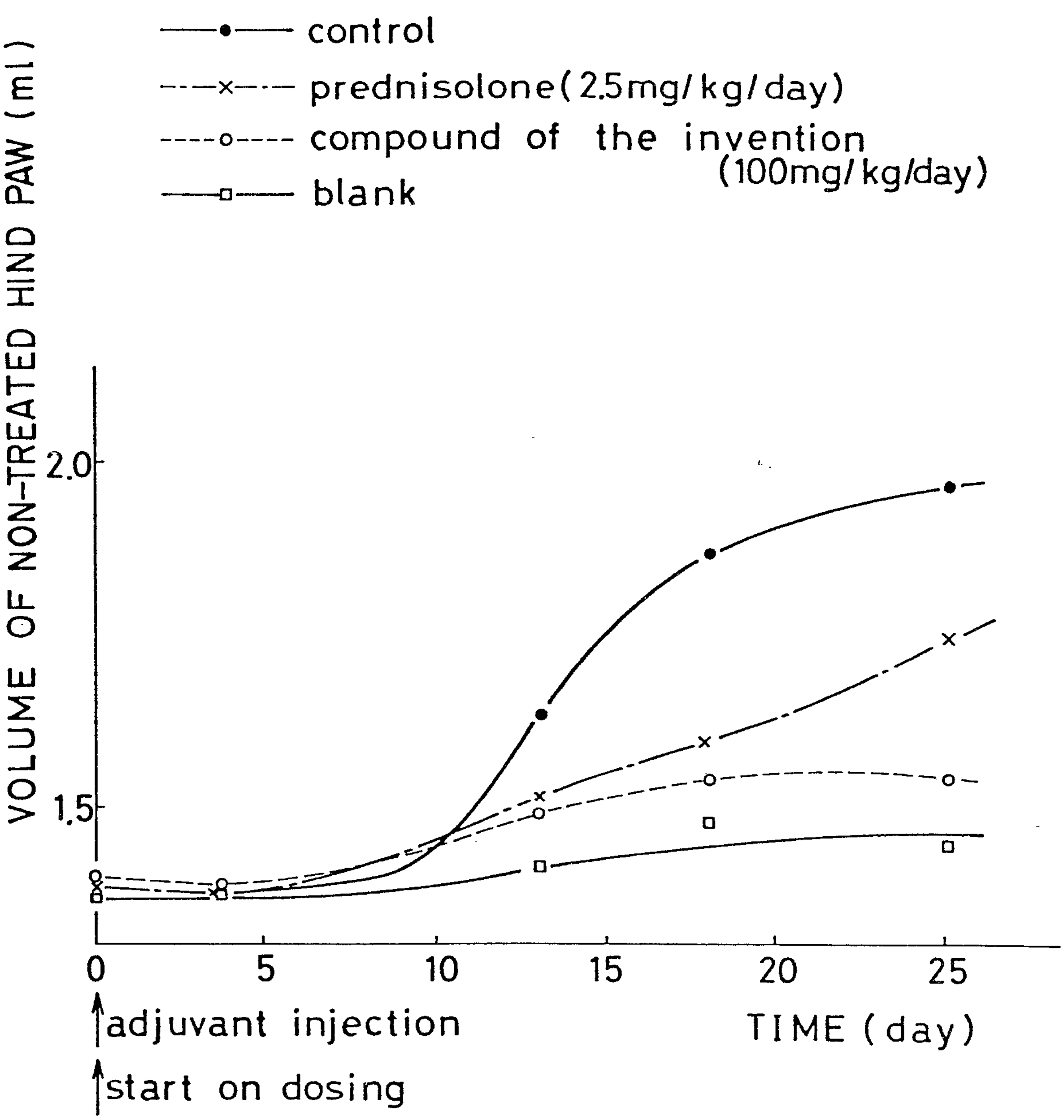
control
prednisolone(2.5mg/kg/day)
compound of the invention (100mg/kg/day)
blank
VOLUME OF NON-TREATED HIND PAW (ml)
2.0
1.5
0
5
10
15
20
25
adjuvant injection
start on dosing
TIME (day)

FIG.6

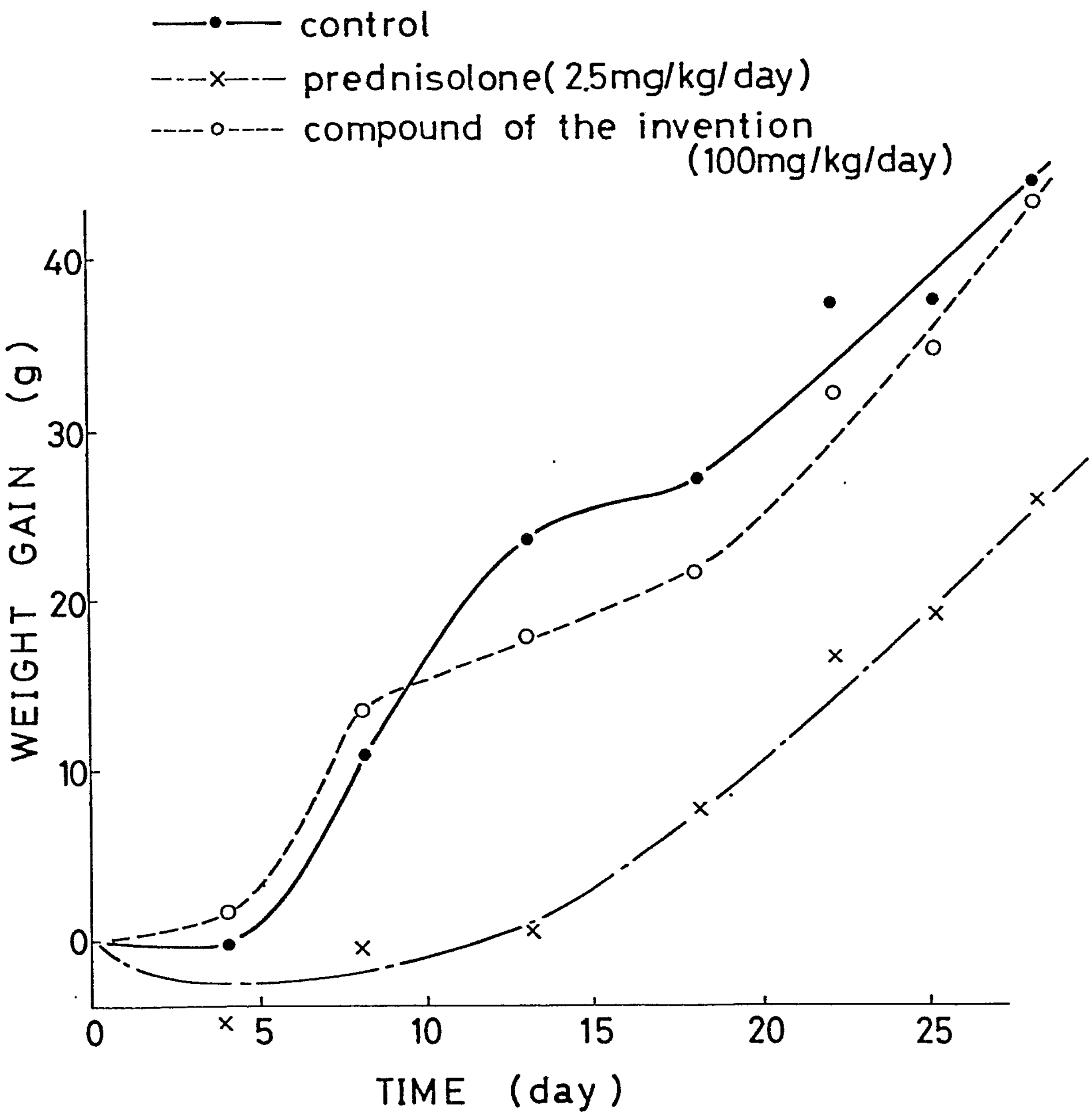
control
prednisolone( 2.5mg/kg/ day)
compound of the invention (100mg/kg/day)
WEIGHT GAIN (g)
40
30
20
10
0
0
5
10
15
20
25
TIME (day)